# EUROPEAN PATENT APPLICATION

(11) **EP 3 798 318 A1**
(43) Date of publication of application: **31.03.2021**
(21) Application number: 19200404.2
(22) Date of filing: 30.09.2019
(51) Int. Cl.: C12Q 1/6806, C12Q 1/6874

(54) **A HIGH THROUGHPUT SEQUENCING METHOD AND KIT**

(71) Applicant: Diagenode S.A., 4102 Seraing (BE)
(72) Inventor: KROONEN, Jérôme, 4000 Liège (BE); SABATEL, Céline, 4550 Villers-le-Temple (BE); BROCART, Gilles, 4130 Esneux (BE)
(74) Representative: Pronovem

(57) **Abstract**

The present invention is in the field of diagnostic and sequencing technologies and is related to a high throughput sequencing method and a kit comprising tools for performing this method, that combine a capture and amplification by switching detection step , preferably the so-called "Capture and Amplification by Tailing and Switching" (CATS) and sequencing technology, preferably the so-called "Nanoballs sequencing" technologies.

## Description

### Field of the invention

The present invention is in the field of diagnostic and sequencing technologies and is related to a high throughput sequencing method and a kit comprising tools for performing this method, that combine a capture and amplification by switching detection step , preferably the so-called "Capture and Amplification by Tailing and Switching" (CATS) and sequencing technology, preferably the so-called "Nanoballs sequencing" technologies.

### Background of the invention

A Capture and Amplification by Switching technology, especially the so-called "Capture and Amplification by Tailing and Switching"(CATS) technology is a ligase-free method to produce DNA libraries for a further sequencing from RNA or DNA and is described in the international patent application WO2015/173402-A1.

There is a need to improve RNA sequencing (or RNA-Seq) which is using the next generation sequencing (NGS) technologies to reveal the presence and quantity of RNA in a biological sample at a given moment, analyzing the continuously changing cellular transcriptome. The Capture and amplification by switching protocols, especially CATS protocol are more efficient for RNA-Seq library creation than protocols using ligase by incorporating adaptors during cDNA synthesis in a single reaction tube. In particular, the CATS technology allows optimal sequencing of sensitive, degraded, cell free RNA (cfRNAs) sequence, plasma derived RNA sequences, non-coding RNA (ncRAS) sequences such as miRNA sequences or long non-coding RNA (IncRNA sequences), exosomal RNA sequences, rare and low input RNA sample, that are efficient markers of different diseases, such as cancers.

Improved sequencing protocols, especially the "Nanoball sequencing" technology disclosed by Drmanac et al (Science 327: 5961, page 78-81 (2010)) require a fragmentation of genomic DNA, wherein individual fragments are used to produce circular DNAs, in which platform specific oligonucleotides adapters separate genomic DNA sequence.

The obtained circular DNAs are amplified to generate advantageously single-stranded concatemers (DNA nanoballs (DNBs) that have a size of about 300 nanometers) that can be immobilized on a substrate at a specific location and that remain separated from each other, because of their negatively charges upon the patterned substrate containing up to 3 billion spots each spot containing one (and only one) DNA nanoball.

### Aims of the invention

The present invention aims to provide a new detection and sequencing method and tools for performing such method that do not present the drawbacks of the method and kit of the state of the art.

A first aim of the present invention is to obtain a method and tools for performing this method that improve the nucleic acids libraries production and sequencing, especially of sensitive, degraded, chemically modified, cell free nucleic acid sequences, especially RNA sequences, possibly obtained from a single cell.

A further aim of the invention is to obtain such method and tools for performing this method that are easy to use, with minimal hands-on time; that are also robust and present an improved sensitivity and excellent reproducibility.

### Definitions.

All literature and similar material cited in the application, including, but no limited to patents, patent applications, scientific articles, books and web pages are expressly incorporated by reference in their entirety to the description of the present invention.

Unless otherwise defined, all terms used in disclosing the invention, including technical and scientific terms, have the meaning as commonly understood by one of ordinary skilled in the art in the invention field.

A used in this description and claims, the singular forms "a", "an", and "the" include singular and plural referents, unless the content of the description clearly dictates otherwise.

The terms "comprising", "comprises" and "composed of" are synonymous to "including" or "containing" and are inclusive and not open ended and do not exclude any additional, non-recited members, elements or methods steps.

The terms "one or more" or "at least one" are clear per se and encompasses a reference to any of these members, which means any two or more of the members and up to all members .

The term "about" as used herein, when referring to a measurable value, such as an amount of a compound, dose, time and the like is meant to encompass 10%, 9%, 8%, 7%, 6%, 5%, 4%, 3%, 2%, 1%, 0.5% or even 0;1% of the specified amount or value.

As used in the description and claims, the terms "nucleic acid(s)" includes polymeric and oligomeric macromolecules, made of DNA (deoxyribonucleic acid) and RNA (ribonucleic acid) known as nucleotides, comprising bases selected from the group consisting of Adenine (A), Thymine (T), Cytosine (C), Guanine (G) and Uracil (U).

The terms "single stranded nucleic acids" (ss nucleic acid) refer to a nucleic acid consisting of only one polynucleotide or oligonucleotide strand. In contrast a "double stranded nucleic acid " (ds nucleic acid) consist of two polynucleotide or oligonucleotide strands wherein the majority of the nucleotides are paired according to known pairing rules.

The terms "genetic amplification" is a biochemical technology used in molecular biology for many years to amplify by primer sequences, a single or few copies of a piece or portion of DNA by replication and copy across several orders of magnitude, generating thousands to millions of copies of a particular DNA sequence. The most known genetic amplification technology is the so called "polymerase Chain Reaction or PCR" as described in US patents 4, 683, 195-B2 and 4, 683, 202- B2 using primers sequences and the heat stable DNA polymerase, such as the Taq polymerase obtained from Bacterium Thermus aquatic allowing thermal cycling.

The term "primer" refers to a oligonucleotide sequence, usually comprising between about 12 nucleotides and about 25 nucleotides, hybridizing specifically to a target sequence of interest and which functions as a substrate onto which nucleotides can be polymerized by a polymerase.

The terms "Template Switch Oligo" or TSO, refer to an oligo that hybridizes to untemplated C nucleotides added by a reverse transcriptase during reverse transcription.

### Summary of the invention

The present invention is related to a high throughput (detection and) sequencing method of a nucleic acid strand sequence as well as tools (preferably included into a kit) for performing this method, this (detection and sequencing) method comprising at least (or consisting of the steps of, preferably the consecutive steps of :
- providing a sample, especially liquid or solid biopsies, such as a blood sample, preferably the plasma, a tissue sample, a fossil, a single cell sample or even targeted compartments of cells (nucleus, endoplasmic reticulum, ...), this sample comprising a native single stranded nucleic acid sequence or native double stranded nucleic acid sequence,
- possibly fragmenting the native single stranded nucleic acid sequence or native double stranded nucleic acid sequence, into smaller nucleic acid sequence fragments
- possibly denaturing the native double strand nucleic acid sequence(s)
- possibly end-repairing the native nucleic acid sequences
- possibly adding at least 5 consecutive nucleotides to the 3-terminus of the native single stranded or native double stranded nucleic acid sequence or their fragments,
- hybridizing a priming oligonucleotide sequence complementary to the added nucleotide sequence and synthesizing a cDNA sequence with a template dependent DNA polymerase to obtain a double stranded nucleic acid sequence,
- hybridizing a template switching oligonucleotide (TSO) to this generated double stranded nucleic acid sequence,
- extending the 3' end of the cDNA strand to synthesize a double stranded nucleic acid sequence, wherein one strand of the nucleic acid sequence comprises the priming oligonucleotide and a cDNA sequence that is complementary to the single stranded nucleic acid sequence and to the template switching oligonucleotide sequence. When the reverse transcriptase reaches the 5'-end of the nucleic acid sequence, it switches the template and continue DNA synthesis over the template-switching oligonucleotide (TSO). The TSO containing three 3'-terminal ribonucleotides x (rX) facilitates the template switching and carry adaptor sequence,
- possibly adding a splint oligo sequence that hybridize to adapter DNA sequences which are ligated to form a circle and adding an exonuclease to remove all remaining single stranded and double stranded DNA products to collect only circular DNA template,
- obtaining DNA nanoballs (DNBs) by performing a rolling circle replication of the synthesized stranded nucleic acid sequence,
- fixing the DNA nanoballs (DNBs) on a patterned array flow cell,
- performing a base sequencing, preferably this base sequencing is obtained by a method selected from the group consisting of synthesis, ligase base sequencing or pyrosequencing, and
- obtaining an identification of each nucleotide of the native nucleic acid sequence, preferably by nanopore sequencing or imaging, more preferably upon a high resolution CCD camera.

In the method of the invention, the synthesized double stranded nucleic acid sequences present a length preferably comprised between about 200 and about 500 nucleotides.

According to the invention, the native single stranded nucleic acid sequence or native double stranded nucleic acid sequence is preferably selected from the group consisting of fragmented and/or bisulfite-converted DNA sequence, mRNA sequence, miRNA sequence small rna sequence, piRNA sequence, bisulfite-converted RNA or a mixture thereof.

In the method according to the invention, the at least 5 consecutive identical nucleotides are preferably selected from the group consisting of ribo-, desoxy-ribonucleotides or didesoxy-ribonucleotides of A, T, C, G or U, that are preferably added by an enzyme selected from the group consisting of a poly(A)-polymerase, poly(U)-polymerase, poly(G)-polymerase, terminal transferase, DNA ligase, RNA ligase and the dinucleotides and the trinucleotides RNA ligases.

Another aspect of the invention concerns an apparatus or a sequencing kit for performing the method of the invention, this kit or apparatus comprising (or consisting of) the following reagents present in suitable vials
- a reagent capable of adding nucleotides to the 3-terminus of a single stranded nucleic acid,
- reagents for a genetic amplification, preferably reagents for performing a PCR amplification a reverse transcriptase enzyme,
- a priming oligonucleotide,
- a template switching oligonucleotide,
- a rolling circle replication enzyme, preferably the Phi 29 DNA polymerase
- possibly reagents for cyclization,
- possibly a patterned flow cell and
- possibly a template independent DNA or RNA polymerase and a blocking nucleotide, such as 3d-NTP, 3-Me-NTP and ddNTP, and
- possibly (written) instructions for performing the method steps of the invention.

In the method, apparatus and kit according to the invention, the priming oligonucleotide preferably comprises the nucleotide sequence disclosed in claims 9 to 12 and claims 19 and 20 of WO2015/173402 incorporated herein by reference.

Advantageously, in the method, apparatus and kit according to the invention, the rolling cycle amplification is obtained by addition of a sufficient amount of the Phi 29 DNA polymerase, this enzyme allowing a production of concatemers or DNA nanoballs (DNBs) into a long single stranded DNA sequence comprising several head-to-tail copies of the circular template, wherein the resulting nanoparticle self assembles into a tight ball of DNA.

This polymerase replicates the looped DNA and when it finishes one circle, it does not stop-it, continues the replication by peeling off its - previously copied DNA. This copying process continues over and over, forming the DNA nanoball this large mass of repeating DNA to be sequenced all connected together.

Preferably, in the method, apparatus and kit according to the invention the patterned array flow cell is a silicon wafer coated with silicon dioxide, titanium, hexamethyldisilazane (HDMS) and a photoresist material and each DNA nanoball selectively binds to the positively-charged aminosilane according to the pattern.

Advantageously, in the method of the invention, the ligase base sequencing is obtained by adding dNTP incorporated by polymerase, each dNTP being preferably conjugated to a particular label or comprises a modification that allows their future detection through a binding with one more labeled antibody(ies) (CooINGS ® technology improved in sensitivity and less costly for obtaining more accurate and longer reads), preferably a label being a fluorophore or dye and possibly containing a termination blocking addition extension, wherein unincorporated dNTPs are washed, wherein image is captured, wherein dye and terminator are preferably cleaved and wherein these steps are repeated until sequencing is complete.

The CoolNGS technology is based the use of multiple fluorescent dye molecules attached to the antibodies providing a higher signal-to-noise ratio and reduced consumption of expensive materials, together with incorporating natural bases with no interference between sequencing cycles.

In addition, in the method of the invention, the added fluorophore is excited with a laser that excites specific wavelength of light and the emission of fluorescence from each DNA nanoball is captured on high resolution CCD camera and wherein the color of each DNA nanoball corresponding to a base to the interrogative position and wherein the computer records the base position information.

A last aspect of the invention concerns the use of the apparatus, the kit or the method according to anyone of the preceding claims, for sequencing or expression analysis, cloning labelling, for the identification of genes or mutation, in personalized medicine, therapy monitoring, prediction, prognosis, early detection of human or animal disease or forensic science, analysis of infectious diseases and genomes of viruses, bacteria, fungi, animals or plant, including their derived cells, characterization of plants, fruits, breeding checks detection of plants or fruits diseases.

## Claims

1. A high throughput sequencing method of a nucleic acid strand sequence comprising the steps of
- providing a sample, single cell sample, comprising a native single stranded nucleic acid sequence or native double stranded nucleic acid sequence,
- possibly fragmenting the native single stranded nucleic acid sequence or native double stranded nucleic acid sequence, into smaller nucleic acid sequence fragments
- possibly denaturing the native double strand nucleic acid sequence(s)
- possibly end-repairing the native nucleic acid sequences,
- hybridizing a priming oligonucleotide sequence complementary to the added nucleotide sequence and synthesizing a cDNA sequence with a template dependent DNA polymerase to obtain a double stranded nucleic acid sequence
- hybridizing a template switching oligonucleotide to the said generated double stranded nucleic acid sequence,
- extending the 3' end of the cDNA strand to synthesize a double stranded nucleic acid sequence, wherein one strand of the nucleic acid sequence comprises the priming oligonucleotide and a cDNA sequence that is complementary to the single stranded nucleic acid sequence and to the template switching oligonucleotide sequence,
- possibly adding a splint oligo sequence that hybridize to adapter DNA sequences which are ligated to form a circle and adding an exonuclease to remove all remaining single stranded and double stranded DNA products to collect only circular DNA template,
- obtaining DNA nanoballs (DNBs) by performing a rolling circle replication of the synthesized double stranded nucleic acid sequence,
- fixing the DNA nanoballs (DNBs) on a patterned array flow cell,
- performing a base sequencing and
obtaining an identification, preferably of each nucleotide of the native nucleic acid sequence, by nanopore sequencing or imaging preferably upon a high resolution CCD camera.

2. The method of claim 1, which comprises the step of adding at least 5 consecutive nucleotides, to the 3-terminus of the native single stranded or native double stranded nucleic acid sequence or their fragments, before the hybridizing step and wherein the at least 5 consecutive nucleotides are selected from the group consisting of ribo-,desoxy-ribonucleotides or didesoxy-ribonucleotides of A, T, C, G or U and are preferably added by an enzyme selected from the group consisting of a poly(A)-polymerase, poly(U)-polymerase, poly(G)-polymerase, terminal transferase, DNA ligase, RNA ligase and the dinucleotides and the trinucleotides RNA ligases

3. The method according to the claim 1 or to the claim 2, the synthesized double stranded nucleic acid sequences present a length comprised between about 200 and about 500 nucleotides.

4. The method according to any one of the preceding claims 1 to 3, wherein the native single stranded nucleic acid sequence or native double stranded nucleic acid sequence is selected from the group consisting of fragmented and/or bisulfite-converted DNA sequence, mRNA sequence, miRNA sequence small RNA sequence, piRNA sequence, bisulfite-converted RNA or a mixture thereof.

5. The method according to any of the preceding claims 1 to 4, wherein the rolling cycle amplification is obtained by addition of a sufficient amount of the Phi 29 DNA polymerase.

6. The method according to any of the preceding claims 1 to 5, wherein the rolling circle replication allows the production of concatemers or DNA nanoballs (DNBs) into a long single stranded DNA sequence comprising several head-to-tail copies of the circular template, wherein the resulting nanoparticle self assembles into a tight ball of DNA.

7. The method according to any one of the preceding claims 1 to 6, wherein the patterned array flow cell is a silicon wafer coated with silicon dioxide, titanium, hexamethyldisilazane (HDMS) and a photoresist material.

8. The method according to any one of the preceding claims 1 to 7, wherein each DNA nanoball selectively binds to the positively-charged aminosilane according to a pattern.

9. The method according to any one of the preceding claims 1 to 8, wherein the ligase base sequencing is obtained by adding dNTP incorporated by polymerase, each dNTP either being modified to be recognized by one or more labelled antibody(ies) or being conjugated to a particular label, preferably a label being a fluorophore and containing a termination blocking addition extension, wherein unincorporated dNTPs are washed, wherein image is captured, wherein dye and terminator are preferably cleaved and wherein these steps are repeated until sequencing is complete.

10. The method according to the claim 9, wherein the added fluorophore is excited with a laser that emits specific wavelength of light and the emission of fluorescence from each DNA nanoball is captured on high resolution CCD camera and wherein the color of each DNA nanoball corresponding to a base to the interrogative position and wherein the computer records the base position information.

11. A sequencing kit comprising
- a reagent capable of adding nucleotides to the 3-terminus of a single stranded nucleic acid,
- an end-repair enzyme,
- reagents for a genetic amplification, preferably a PCR genetic amplification,
- a reverse transcriptase enzyme,
- a priming oligonucleotide,
- a template switching oligonucleotide and
- a rolling circle replication enzyme

12. The kit of claim 11, further comprising a patterned flow cell, being preferably a silicon wafer coated with silicon dioxide, titanium, hexamethyldisilazane (HDMS) and a photoresist material

13. The kit according to any one of the claim 11 or claim 12, wherein the reagent is a template independent DNA or RNA polymerase and a blocking nucleotide, such as 3d-NTP, 3-Me-NTP and ddNT.

14. The kit according to any one of the preceding claims 11 to 13, wherein the rolling circle replication enzyme is the Phi 29 DNA polymerase.

15. Use of the kit or the method according to anyone of the preceding claims, for sequencing or expression analysis, cloning labelling, for the identification of genes or mutation, in personalized medicine, therapy monitoring, prediction, prognosis, early detection of human or animal disease or forensic science, analysis of infectious diseases and genomes of viruses, bacteria, fungi, animals or plant, including their derived cells, characterization of plants, fruits, breeding checks detection of plants or fruits diseases.
